# EUROPEAN PATENT SPECIFICATION

(11) **EP 1 267 763 B1**
(45) Date of publication and mention of the grant of the patent: **29.11.2006**
(21) Application number: 01900840.8
(22) Date of filing: 03.01.2001
(51) Int. Cl.: A61F 13/00, A61F 13/02

(54) **NOVEL WOUND DRESSING, PROCESS OF MANUFACTURE AND USEFUL ARTICLES THEREOF**
WUNDVERBAND, HERSTELLUNGSVERFAHREN UND DARAUS ERSTELLBARE GEGENSTÄNDE
NOUVELLE MATIERE DE PANSEMENT, SON PROCEDE DE FABRICATION ET ARTICLES UTILES PRODUITS A PARTIR DE LADITE MATIERE DE PANSEMENT

(30) Priority: 03.01.2000 US 174477 P
(43) Date of publication of application: 02.01.2003
(73) Proprietor: Biomed Sciences Inc., Allentown, PA 18101 (US)
(72) Inventor: DILLON, Mark, E., Menungie, PA 18062 (US)
(74) Representative: Effert, Bressel und Kollegen
(86) International application number: PCT/US2001/000130
(87) International publication number: WO 2001/049228

(56) References cited:
- EP-A- 0 254 493
- US-A- 3 949 742
- US-A- 4 051 848
- US-A- 5 147 338
- US-A- 5 653 699

## Description

### BACKGROUND OF THE INVENTION

1. Field of the Invention This invention relates to a novel wound dressing design. Particularly, this invention relates to a wound dressing which incorporates two distinct layers, each providing useful features and together providing a novel method of managing a variety of wound types. Ease of use, patient comfort and the cost of care are improved.

### 2. Description of the Prior Art

In the field of woundcare there exist several general categories of commonly used dressings. Some dressings aggressively adhere to the wound surface. For example, conventional gauze integrates into the wound as healing occurs and eschar forms on the wound surface. Other types of dressings are designed to adhere to the surrounding intact tissue around the wound site, but not directly to the wound. Examples of this type of dressing include polyurethane films coated with pressure sensitive adhesive. Other types of dressings are designed to be substantially nonadherent. Examples of this type include polyethylene oxide hydrogels, and particularly the material described in U.S. Patent number 4,832,009. The latter example is a dressing made from an interpenetrating polymer network ("IPN") of polytetrafluoroethylene and silicone, and is presently marketed by Bio Med Sciences, Inc. of Allentown, PA as Silon-TSR^{®} Temporary skin Replacement. Bach type of dressing has its advantages and disadvantages, and is indicated for certain wound conditions and user preferences.

Typical type wound dressings are for example described in U.S. Patent number 5,147,338 U.S. Patent number4,051,848, EP 0 254 493 A1, U.S. Patent number 5,653,699 and U.S. Patent number 3,949,742.

U.S. Patent number 5,147,338 relates to an absorptive wound dressing suitable for the use on burns or other wounds. The wound dressing has a reduced tendency to adhere to the wound and can act as a bacterial barrier. The wound dressing comprises a wound facing layer, an intermediate absorbent layer and an outer layer. The wound facing layer comprises a conformable elastomeric apertured film, made from thermoplastic polyurethanes and polybutadienes for example. The intermediate absorbent layer comprises conformable hydrophilic foam. The outer layer comprises a continuous moisture vapor transmitting conformable film.

U.S. Patent number 4,051,848 relates to a wound dressing which acts as a temporary synthetic skin. It is comprised of an outer component and of an inner component. The outer component is a microporous membrane permeable to gases but impermeable to micro-organisms. The inner component is a three dimensional matrix structure made of at least two substantially coextensive layers of knitted fabric each bound into the other at closely spaced points to retain stretchability. Each fabric layer is knitted from threads spaced from each other at such a distance that cellular elements from the tissue denuded from skin may invade the dressing to promote healing. The outer component microporous membrane may be a polytetrafluoroethylene film, vulcanized latex, silicone rubber or polypropylene film. The threads from which the fabric is knitted may be of any fine denier natural or synthetic fiber material such as polyamides, rayon, dacron, polyester, cotton, silk, cat gut, polyglycolic acid and certain further trade name materials. The inner component serves as a scaffolding layer to adhere to the denuded tissue and which the cellular elements of the wound can invade.

U.S. Patent number 5,653,699 relates to a sheet form composite as a spyrosorbent wound dressing. The sheet-form composite includes a hydrophilic, microporous, monolithic film layer. This film layer is laminated to a hydrophilic exudate transport layer. The hydrophilic exudate transport layer is constituted by physiologically tolerable material that is hydratable or swellable by, but not soluble in, the wound exudate. It can be comprised of one or more of hydrocolloids, gels (hydrogels or hydroalcoholic gels), foams, textiles (woven or nonwoven), membranes (microporous or macroporous) and hydrophilic adhesives (pressure-sensitive or biadhesive). The film layer may be comprised of segmented polyurethane, which is liquid impermeable but water vapor permeable. In use the exudate transport layer is applied in contact with the wound. An adhesive layer may be included in at least the border portion for securement of the dressing.

U.S. Patent number 3,949,742 relates to a medical dressing which is adapted to perform as a synthetic skin for the therapy and protection of skin wounds. The medical dressing comprises a thin layer of non-porous segmented polyurethane cohesively secured to a thin layer of thrombogenic reticulated foam, which latter is non-porous. It allows oxygen and water vapor to pass there through. The foam layer is a thin facing layer and the elastomeric layer is a backing layer. This backing layer is preferably a thin film of segmented polyurethane resins. The facing layer may contain a drug such as an antibiotic, proteolytic enzyme, topical anesthetic and the like

EP 0 254 493 A1 relates to a wound dressing which comprises an adhesive-free central area which is non-adherent to wounds. The central area comprises a layer of synthetic polymer. This synthetic polymer may preferably form a continuous film of a hydrophilic polymer and more particularly of hydrophilic polyurethane. An absorbent pad may be present at the non-wound facing side of the central area, to absorb any fluid which may pass through it. The synthetic polymer layer in the central area may be a film, more specifically a moisture vapor permeable film including those formed from polyurethane, polyvinyl alcohol, cellulose derivatives, elastomeric polyester, polyether polyamide and the like. The absorbent pad on the non-wound facing side may be formed from materials including gauze, cellulose pads, synthetic polymer foam and other conventional body fluid absorbing materials.

There are a wide variety of wound types. Wounds can be categorized as chronic or acute. Examples of chronic wounds include venous stasis ulcers, decubitus ulcers and diabetic ulcers. Examples of acute wounds include burns, skin graft donor sites, skin graft recipient sites, abrasions and the like. The features required for the proper performance of a wound dressing depend on the wound type as well as the location of the wound on the body. For example, non-adherent films minimize disruption of fragile skin during dressing changes, but are not always applicable because of difficulties in keeping the dressing in position. This is particularly a challenge for skin graft donor sites on the back or buttocks of a patient, where ordinary movement and contact with bedding can easily dislodge the dressing. As a result, adhesive dressings are typically used for this type of wound. An additional example includes the use of absorbent dressings on chronic wounds. Chronic wounds tend to produce copious amounts of exudate which makes the use of thin film dressings difficult since these dressings are generally poor at managing wound fluid.

Even the same wound may require different dressings at different stages of the healing process. A venous stasis ulcer will produce copious amounts of exudate in the early stages of healing. Hydrocolloid dressings are often used on these wounds because of their high absorption capabilities. But as a wound of this type heals, the fragile epithelium can easily be damaged during dressing changes, so a non-adherent dressing may be substituted later in the healing process even if it is not as absorbent.

Bio Med Sciences, Inc. manufactures a thin-film non-adherent dressing made from an interpenetrating polymer network ("IPN") of polytetrafluoroethylene and silicone (Silon-TSR^{®}). The IPN film is flexible and thin (50 microns), thereby providing transparency and good conformity to wound contours. Small fenestrations are cut through the film so that wound fluid can wick away from the wound surface and be collected in a secondary dressing such as gauze. The outer gauze may be changed as required, but the IPN dressing may be left in place until the wound heals or for up to 10 days.

The IPN dressing is well-suited for applications such as laser resurfacing, which is a cosmetic surgery procedure almost exclusively performed on the face. The product's non-adherent and transparent properties provide clinical advantages during the healing process. This product, however, does not perform as well on certain other types of wounds, such as skin graft donor sites and many types of chronic wounds. The non-adherent character of the product is problematic for application on any part of the body where shear forces, such as contact with bedding or other surfaces, may cause the dressing to roll-up or slide off of the wound. This difficulty is particularly acute on lower limbs where the general shape tends to be somewhat conical thereby causing the dressing to slide distally.

The IPN dressing manufactured by Bio Med Sciences, Inc. provides desirable properties with respect to a conformable non-adherent surface for wound coverage. These features, however, have proved to be problematic with respect to maintaining wound coverage and avoiding dressing roll-up and slippage.

### SUMMARY OF THE INVENTION

In an effort to mitigate said problematic characteristics, I have unexpectedly created a dressing with a unique dual-purpose design.

The new dressing comprises a thin layer (50 microns) of the IPN material laminated to a polyurethane foam of approximately 1,500 microns in thickness. This construction has the effect of providing a greater cross-sectional thickness, which tends to be more resistant to roll-up, wrinkling and slippage.

By applying the dressing to the wound site with the IPN surface against the wound surface, the non-adherent advantages of the IPN material are preserved. At the same time, however, the foam layer minimizes any tendency for the dressing to slip, roll-up or wrinkle. Fenestrations are still cut through the IPN material and the foam passes wound exudate through to a secondary dressing.

Unexpectedly, I have discovered that the dressing of this invention is also useful for woundcare when used "up-side-down" with the foam layer against the wound instead of the IPN layer. This serves to provide a dressing with a higher level of surface adhesion but otherwise similar features, wound fluid is still wicked from the wound surface to a secondary dressing and slippage or roll-up are still minimized.

This invention provides a single dressing that can offer disparate wound healing features depending on its orientation on the wound surface. This is useful for broadening the range of clinical applications for which either the membrane (IPN) material or the foam layer could be used individually. This is true for different clinical cases or for the same case at different stages of the healing process.

While the two opposite approaches to wound healing (adhesive/non-adhesive) are commonly found in the field, no product combines these two features in a single dressing by means of simply using it one side up or the other. This provides great utility in the field where the number of products stocked is always minimized to reduce inventory costs. In addition, cost effectiveness is promoted due to consolidated manufacturing and distribution operations. Most importantly, this invention provides a unique-dual-purpose dressing for a wide variety of wound types.

### BRIEF DESCRIPTION OF THE DRAWINGS

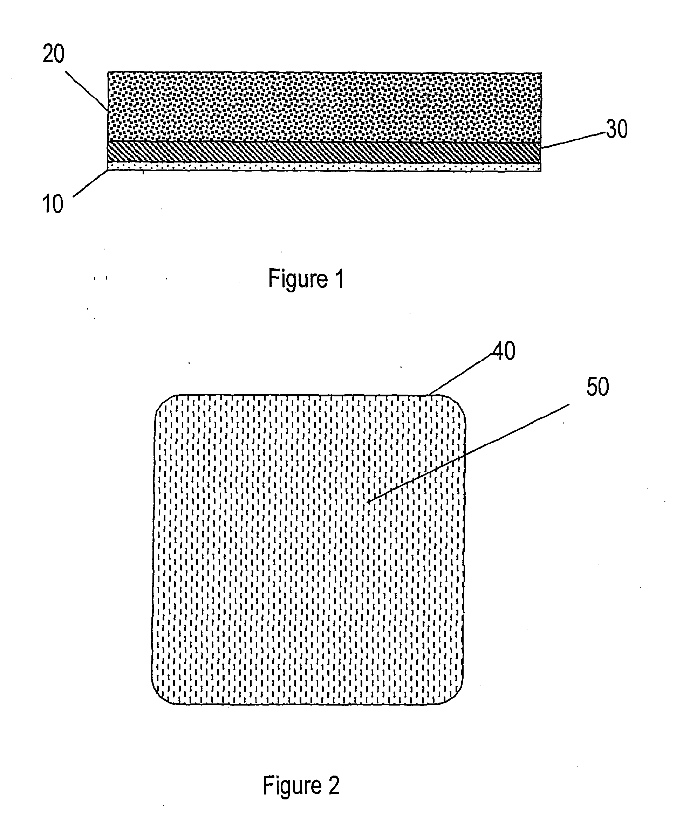

Figure 1 shows a cross-sectional view of a preferred embodiment of this invention. The IPN material 10 is bonded to a foam layer 20 by means of silicone elastomer 30.
Figure 2 shows a plan view of a dressing 40 cut from the material of this invention. Fenestrations 50 are cut through the IPN film to provide a means for managing wound exudate.

### DETAILED DESCRIPTION OF PREFERRED EMBODIMENTS

Turning to the drawings, there is shown the inventive new dressing which comprises a thin layer (50 microns) of IPN material 10 laminated to a polyurethane foam 20 of approximately 1,500 microns in thickness. Preferably, a silicone elastomer 30 is used to bond the IPN material 10 to the polyurethane foam 20. Fenestrations 50 are cut through the IPN film to provide a means for managing wound exudate.

The following examples are not intended to be limiting, as minor variations on these designs and processes would be obvious to those skilled in the art. Likewise, it is believed that other materials could be used to achieve the same dressing design.

### Example 1:

A continuous sheet of polydimethylsiloxane and polytetrafluoroethylene IPN was manufactured according to established methods. The film measured approximately 50 microns in thickness. The IPN film was then passed through a knife-over-roll assembly and coated with approximately 200 microns of liquid silicone rubber MDX4-4210 from Dow Corning Corporation of Midland, MI. Soon after the silicone rubber was applied to the IPN material, an open-cell hydrophilic foam (Amrel^{®} Medical Foam from Rynel Limited, Inc. of Boothbay, ME) was laid onto the uncured silicone rubber and the laminate was passed through a tunnel style oven at approximately 150°C for approximately 6 minutes. The resultant material was then fed through a rotary die-cutting apparatus to cut individual dressings from the sheet and to create fenestrations in the IPN film.

### Example 2:

The process of Example 1 was repeated with a pigment added to the liquid silicone rubber prior to the lamination process. A blue silicone-based ink (product code R1008-7 from Nusil Technology of Carpinteria, CA) was mixed into the MDX4-4210 at a concentration of 4 percent by weight. Since the IPN material is transparent and the foam is opaque, the blue pigment imparted a soft blue coloration to one side of the dressing. This serves as a visual indicator for differentiating one side of the dressing from the other in the field.

## Claims

1. A dual-purpose wound dressing capable of providing disparate wound healing characteristics to a wound depending on which side of the wound dressing contacts the wound, comprising
a multilayered composite structure, the multilayered composite structure having a first wound contacting side having an outer wound contacting surface that has wound healing characteristics and a second wound contacting side having an outer wound contacting surface that has wound healing characteristics different from the wound healing characteristics of the outer surface of the first side of the composite structure, the multilayered structure including
a first layer forming the first wound contacting side of the composite structure, the first layer being a membrane layer, and
a second layer forming the second wound contacting side of the composite structure, the second layer being a foam layer.

2. The wound dressing of claim 1, further comprising an adhesive layer for joining the first and second layers together.

3. The wound dressing of claims 1 or 2, wherein the foam layer comprises a polyurethane material.

4. The wound dressing of claims 1-3, wherein the membrane layer comprises a silicone-containing compound.

5. The wound dressing of any of claims 2-4, wherein the adhesive layer comprises a silicone compound.

6. The wound dressing of any of claims 1-5, wherein the first layer comprises an interpenetrating polymer network of polytetrafluoroethylene and silicone.

7. The wound dressing of any of claims 1-6, further comprising a pigment for imparting a discernable color to one side of the composite structure.

8. The wound dressing of any of claims 1 or 3-7, further comprising an adhesive layer for joining the first and second layers together, the first layer being substantially transparent, the second layer being substantially opaque, and the adhesive layer containing a pigment for imparting a discernable color to the first layer of the wound dressing.

9. The wound dressing of any of claims 1-8, further including fenestrations formed in the first layer.

10. The wound dressing of any of claims 2 or 8, the adhesive layer being a silicone elastomer.

11. The wound dressing of any of claims 1-10, the first layer being about 50 microns thick and the second layer being about 1500 microns thick.

12. The wound dressing of any of claims 1-11, the wound dressing having a cross-section of such thickness as to be resistant to roll-up, wrinkling and slippage.

13. A method of manufacturing a dual-purpose wound dressing capable of providing disparate wound healing characteristics to a wound depending on which side of the wound dressing contacts the wound, comprising (1) producing a thin film membrane layer, (2) passing said thin film membrane layer through a coating assembly and depositing a layer of an adhesive substance, and (3) causing a foam material to make intimate contact with said adhesive substance to form a multilayered composite wound dressing having a first wound contacting side and a second wound contacting side, the first wound contacting side of the multilayered wound dressing being formed by the thin film membrane layer and the second wound contacting side of the multilayered wound dressing being formed by the foam material.

14. The method of claim 13, further including cutting the wound dressing into smaller sized wound dressings.

15. The method of any of claims 13 or 14, further including creating fenestrations in the membrane layer.

16. The method of any of claims 13, 14 or 15, the adhesive substance having a pigment mixed therein.

## Patentansprüche

1. Zweifach verwendbarer Wundverband, der geeignet ist, unterschiedliche Wundheilungseigenschaften an einer Wunde in Abhängigkeit von der Seite, mit der der Wundverband die Wunde berührt, vorzusehen, umfassend
eine mehrlagige Verbundstruktur, wobei die mehrlagige Verbundstruktur eine erste die Wunde berührende Seite, die eine äußere die Wunde berührende, wundheilende Eigenschaften aufweisende Oberfläche hat, und eine zweite die Wunde berührende Seite aufweist, die eine äußere die Wunde berührende Oberfläche hat, die wundheilende Eigenschaften aufweist, die von den wundheilenden Eigenschaften der äußeren Oberfläche der ersten Seite der Verbundstruktur verschieden ist, wobei die mehrlagige Struktur aufweist:
eine erste Schicht, die die erste die Wunde berührende Seite der Verbundstruktur bildet, wobei die erste Schicht eine Membranschicht ist, und
eine zweite Schicht, die die zweite die Wunde berührende Seite der Verbundstruktur bildet, wobei die zweite Schicht eine Schaumstoffschicht ist.

2. Wundverband nach Anspruch 1, der außerdem eine Haftvermittlerschicht zum Verbinden der ersten Schicht und der zweiten Schicht miteinander aufweist.

3. Wundverband nach den Ansprüchen 1 oder 2, wobei die Schaumstoffschicht ein Polyurethanmaterial enthält.

4. Wundverband nach den Ansprüchen 1 bis 3, wobei die Membranschicht eine Silikon enthaltende Verbindung enthält.

5. Wundverband nach einem der Ansprüche 2 bis 4, bei dem die Haftvermittlerschicht eine Silikonverbindung enthält.

6. Wundverband nach einem der Ansprüche 1 bis 5, bei dem die erste Schicht ein sich gegenseitig durchdringendes Polymernetzwerk von Polytetrafluorethylen und Silikon enthält.

7. Wundverband nach einem der Ansprüche 1 bis 6, der außerdem ein Pigment enthält, dass einer Seite der Verbundstruktur eine wahrnehmbare Farbe verleiht.

8. Wundverband nach einem der Ansprüche 1 und 3 bis 7, der außerdem eine Haftvermittlerschicht zum Verbinden der ersten Schicht und der zweiten Schicht miteinander umfasst, wobei die erste Schicht im Wesentlichen transparent ist und die zweite Schicht im Wesentlichen undurchsichtig ist und wobei die Haftvermittlerschicht ein Pigment enthält, das der ersten Schicht des Wundverbandes eine wahrnehmbare Farbe verleiht.

9. Wundverband nach einem der Ansprüche 1 bis 8, der außerdem ein in der ersten Schicht gebildetes Fensterwerk aufweist.

10. Wundverband nach einem der Ansprüche 2 oder 8, bei dem die Haftvermittlerschicht ein Silikonelastomer ist.

11. Wundverband nach einem der Ansprüche 1 bis 10, bei dem die erste Schicht etwa 50 µm dick ist und die zweite Schicht etwa 1500 µm dick ist.

12. Wundverband nach einem der Ansprüche 1 bis 11, wobei der Wundverband einen Querschnitt mit einer derartigen Dicke hat, dass er gegenüber Aufrollen, Faltenbildung und Schlupf widerstandsfähig ist.

13. Verfahren zum Herstellen eines zweifach verwendbaren Wundverbandes, der geeignet ist, unterschiedliche Wundheilungseigenschaften an einer Wunde in Abhängigkeit von der Seite, mit der der Wundverband die Wunde berührt, vorzusehen, umfassend
(1) Anfertigen einer Dünnschicht-Membran,
(2) Passieren der Dünnschicht-Membran durch eine Beschichtungsanordnung und Abscheiden einer Schicht eines Klebstoffes, und
(3) Veranlassen, dass ein Schaumstoffmaterial in engen Kontakt mit dem Klebstoff tritt, so dass ein mehrlagiger Verbund-Wundverband gebildet wird, der eine erste die Wunde berührende Seite und eine zweite die Wunde berührende Seite aufweist, wobei die erste die Wunde berührende Seite des mehrlagigen Wundverbandes durch die Dünnschicht-Membran gebildet wird und die zweite die Wunde berührende Seite des mehrlagigen Wundverbandes durch das Schaumstoffmaterial gebildet wird.

14. Verfahren nach Anspruch 13, bei dem der Wundverband außerdem in Wundverbände mit geringerer Größe geschnitten wird.

15. Verfahren nach einem der Ansprüche 13 oder 14, bei dem außerdem ein Fensterwerk in der Membran erzeugt wird.

16. Verfahren nach einem der Ansprüche 13, 14 oder 15, bei dem der Klebstoff ein hinein gemischtes Pigment enthält.

## Revendications

1. Pansement à double fonction pouvant fournir des caractéristiques disparates de cicatrisation d'une blessure à une blessure en fonction du côté avec lequel le pansement est en contact avec la blessure, comprenant
une structure composite à couches multiples, la structure composite à couches multiples comportant un premier côté en contact avec la blessure comprenant une surface externe de contact avec la blessure qui présente des caractéristiques de cicatrisation de blessure et un second côté en contact avec la blessure comprenant une surface externe de contact avec la blessure qui présente des caractéristiques de cicatrisation de la blessure différentes des caractéristiques de cicatrisation de la blessure de la surface externe du premier côté de la structure composite, la structure à couches multiples comprenant
une première couche formant le premier côté en contact avec la blessure de la structure composite, la première couche étant une couche de membrane, et
une seconde couche formant le second côté en contact avec la blessure de la structure composite, la seconde couche étant une couche de mousse.

2. Pansement selon la revendication 1, comprenant en outre une couche adhésive pour joindre ensemble les première et seconde couches.

3. Pansement selon la revendication 1 ou 2, dans lequel la couche de mousse comprend du polyuréthane.

4. Pansement selon les revendications 1 à 3, dans lequel la couche de membrane comprend un composé contenant de la silicone.

5. Pansement selon l'une quelconque des revendications 2 à 4, dans lequel la couche adhésive comprend un composé siliconé.

6. Pansement selon l'une quelconque des revendications 1 à 5, dans lequel la première couche comprend un alliage IPN de polytétrafluoroéthylène et de silicone.

7. Pansement selon l'une quelconque des revendications 1 à 6, comprenant en outre un pigment destiné à conférer une couleur discernable à un côté de la structure composite.

8. Pansement selon l'une quelconque des revendications 1 ou 3 à 7, comprenant en outre une couche adhésive destinée à joindre ensemble les première et seconde couches, la première couche étant sensiblement transparente, la seconde couche étant sensiblement opaque, et la couche adhésive contenant un pigment destiné à conférer une couleur discernable à la première couche du pansement.

9. Pansement selon l'une quelconque des revendications 1 à 8, comprenant en outre des perforations formées dans la première couche.

10. Pansement selon l'une quelconque des revendications 2 à 8, la couche adhésive étant un élastomère de silicone.

11. Pansement selon l'une quelconque des revendications 1 à 10, la première couche étant épaisse d'environ 50 microns et la seconde couche étant épaisse d'environ 1 500 microns.

12. Pansement selon l'une quelconque des revendications 1 à 11, le pansement ayant une coupe transversale d'une épaisseur telle qu'il est résistant à l'enroulement, au plissement et au glissement.

13. Procédé de fabrication d'un pansement à double fonction pouvant fournir des caractéristiques disparates de cicatrisation de blessure à une blessure en fonction du côté avec lequel le pansement est en contact avec la blessure, comprenant
(1) produire une couche de membrane à couche mince,
(2) faire passer ladite couche de membrane à couche mince par un ensemble de revêtements et déposer une couche d'une substance adhésive, et
(3) amener une mousse à être en contact intime avec ladite substance adhésive pour former un pansement composite à couches multiples comportant un premier côté en contact avec la blessure et un second côté en contact avec la blessure, le premier côté en contact avec la blessure du pansement à couches multiples étant formé par la couche de membrane à couche mince et le second côté en contact avec la blessure du pansement à couches multiples étant formé par la mousse.

14. Procédé selon la revendication 13, comprenant en outre le découpage du pansement en pansements de taille plus petite.

15. Procédé selon l'une quelconque des revendications 13 ou 14, comprenant en outre la création de perforations dans la couche de membrane.

16. Procédé selon l'une quelconque des revendications 13, 14 ou 15, la substance adhésive comportant un pigment mélangé dans celle-ci.
